(19) Europäisches Patentamt
European Patent Office
Office européen des brevets

(11) Veröffentlichungsnummer: **0 133 918**
**A1**

(12) # EUROPÄISCHE PATENTANMELDUNG

(21) Anmeldenummer: **84107710.0**

(22) Anmeldetag: **03.07.84**

(51) Int. Cl.⁴: **C 07 C 127/19**, A 01 N 47/30

(30) Priorität: **06.07.83 DE 3324244**

(43) Veröffentlichungstag der Anmeldung: **13.03.85**
**Patentblatt 85/11**

(84) Benannte Vertragsstaaten: **BE CH DE FR GB IT LI NL**

(71) Anmelder: **BASF Aktiengesellschaft,**
**Carl-Bosch-Strasse 38, D-6700 Ludwigshafen (DE)**

(72) Erfinder: **Becker, Rainer, Dr., Im Haseneck 22,**
**D-6702 Bad Duerkheim (DE)**
Erfinder: **Schirmer, Ulrich, Dr., Berghalde 79,**
**D-6900 Heidelberg (DE)**
Erfinder: **Theobald, Hans, Dr., Queichstrasse 6,**
**D-6703 Limburgerhof (DE)**
Erfinder: **Wuerzer, Bruno, Dr., Ruedigerstrasse 13,**
**D-6701 Otterstadt (DE)**

(54) **Harnstoffderivate, Verfahren zu ihrer Herstellung und ihre Verwendung zur Bekämpfung unerwünschten Pflanzenwuchses.**

(57) Die Erfindung betrifft Harnstoffderivate der Formel

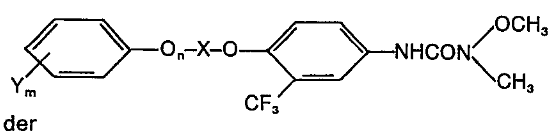

in der

X eine gegebenenfalls substituierte Alkylenkette, Alkenylenkette oder Alkinylenkette, Y Wasserstoff, Alkyl, Halogen, Alkoxy oder Halogenalkyl, m 1 bis 3 und n 0 oder 1 bedeuten, Verfahren zu ihrer Herstellung und ihre Verwendung zur Bekämpfung unerwünschten Pflanzenwuchses.

EP 0 133 918 A1

Harnstoffderivate, Verfahren zu ihrer Herstellung und ihre Verwendung zur Bekämpfung unerwünschten Pflanzenwuchses

Die Erfindung betrifft Harnstoffderivate, Verfahren zu ihrer Herstellung, Herbizide, die diese Verbindungen als Wirkstoffe enthalten, sowie Verfahren zur Bekämpfung unerwünschten Pflanzenwuchses mit diesen Wirkstoffen.

Es ist bekannt, daß N-[4-Benzyloxyphenyl-]-N'-methyl-N'-methoxyharnstoffe (DE-OS 27 11 230; DE-OS 28 69 035) sowie N-[4-Benzyloxy-3-trifluormethylphenyl]- bzw. N-[4-Phenethoxy-3-trifluormethylphenyl]-N'N'-dialkylharnstoffe (US-PS 4 289 903) herbizid wirksam sind.

Es wurde gefunden, daß Harnstoffderivate der Formel

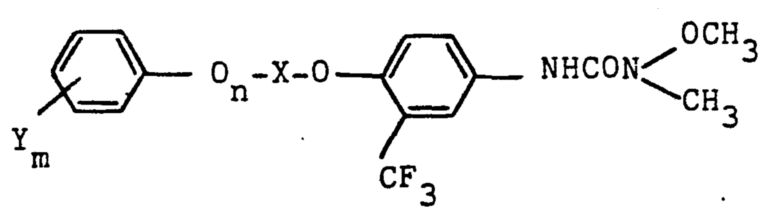

(I),

in der

X eine gegebenenfalls durch $C_1$-$C_4$-Alkyl substituierte $C_1$-$C_4$-Alkylenkette, $C_2$-$C_4$-Alkenylenkette oder $C_2$-$C_4$-Alkinylenkette,

Y Wasserstoff, $C_1$-$C_4$-Alkyl, Halogen, $C_1$-$C_4$-Alkoxy oder $C_1$-$C_4$-Halogenalkyl,

m 1 bis 3 und

n 0 oder 1 bedeuten,

eine herbizide Wirkung haben und für Kulturpflanzen selektiv sind.

In der Formel I bedeuten X eine gegebenenfalls durch $C_1$-$C_4$-Alkyl substituierte $C_1$-$C_4$-Alkylen-, $C_2$-$C_4$-Alkenylen- oder $C_2$-$C_4$-Alkinylenkette, wie Methylen, Dimethylen, Trimethylen, Tetramethylen, Methylmethylen, Methyldimethylen, n-But-2-enylen, n-But-2-in-ylen, Y Wasserstoff, Halogen, $C_2$-$C_4$-Alkyl, $C_1$-$C_4$-Alkoxy oder $C_1$-$C_4$-Halogenalkyl, wie Chlor, Fluor, Brom, Methyl, Ethyl, Isopropyl, Trifluormethyl, Methoxy und m 1, 2 oder 3 Bevorzugte Harnstoffderivate sind solche, bei denen X eine $C_1$-$C_4$-Alkylenkette, Y Wasserstoff und n 0 bedeuten.

Man erhält die Verbindungen der Formel I durch Umsetzung der Halogenverbindungen II

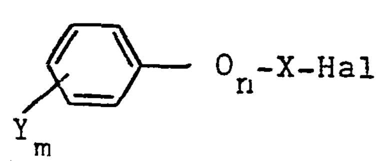

(II),

in der X, Y, m und n die obengenannten Bedeutungen haben und Hal für Halogen steht, mit N-(4-Hydroxy-3-trifluormethylphenyl)-N'-methyl-N'-methoxyharnstoff in Gegenwart eines inerten Lösungsmittel bei einer Temperatur zwischen 0 und 150°C, vorzugsweise zwischen 20 und 80°C.

Die Umsetzung wird zweckmäßigerweise in Gegenwart einer Base durchgeführt, wobei die Menge an Base, bezogen auf die Verbindung II, 1,0 bis 1,5 Mol beträgt. Geeignete Basen sind Alkalicarbonate, Alkalihydrogencarbonate und Alkalihydroxide, wie Natriumcarbonat, Kaliumcarbonat, Natriumhydrogencarbonat, Natriumhydroxid oder Kaliumhydroxid.

Als Lösungsmittel kommen polare organische Lösungsmitte, wie Aceton, Acetonitril, Dimethylformamid, Dimethylsulfoxid, oder Wasser in Betracht. Wird die Reaktion in wäßrigem Medium durchgeführt, so wird zweckmäßigerweise ein Phasentransferkatalysator, z.B. Tetrabutylammoniumjodid, hinzugefügt. Die Menge dieses Katalysators beträgt etwa 0,01 bis 0,1 Mol, bezogen auf 1 Mol an Verbindung II.

Die Reaktionskomponenten werden in äquimolarem Verhältnis umgesetzt. Ein Überschuß der einen oder anderen Komponente stört nicht.

Weiterhin erhält man die Harnstoffderivate der Formel I durch Umsetzung von Hydroxylverbindungen der Formel

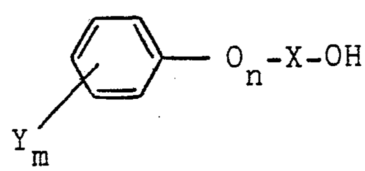 (III),

in der X, Y, m und n die obengenannten Bedeutungen haben, mit einer Halogenverbindung der Formel

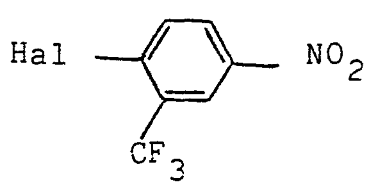 (IV),

in der Hal Halogen bedeutet, zu einer Verbindung der Formel

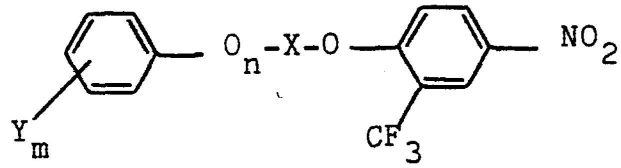 (V),

in der X, Y, m und n die obengenannten Bedeutungen haben, die dann in an sich üblicher Weise zur entsprechenden Aminoverbindung reduziert und durch Umsetzung mit einem N-Methyl-N-methoxy-carbaminsäurehalogenid in ein Harnstoffderivat der Formel I überführt werden kann.

Die Reduktion der Verbindungen der Formel V kann auf üblichem Wege mit beispielsweise Wasserstoff/Katalysator oder $SnCl_2$ oder Eisenpulver durchgeführt werden.

Die Verbindungen der Formel V können durch Umsetzung mit N-Methyl-N-methoxy-carbaminsäurehalogenid, insbesondere dem Chlorid oder Bromid, oder mit Phosgen in das entsprechende Isocyanat und anschließend mit Methyl-Methoxyamin in die Harnstoffderivate der Formel I überführt werden.

Die Verbindungen der Formel V lassen sich weiterhin durch Umsetzung der Halogenverbindungen der Formel II mit 4-Nitro-3-trifluormethyl-phenol herstellen.

Die Bedingungen für die Umsetzung der Verbindungen III mit den Verbindungen IV entsprechen den oben beschriebenen für die Reaktion von II mit N-(4-Hydroxy-3-trifluormethyl-phenyl)-N'-methyl-N'-methoxy-harnstoff.

Die folgenden Beispiele erläutern die Durchführung der erfindungsgemäßen Verfahren. Gewichtsteile verhalten sich zu Volumenteilen wie Kilogramm zu Liter.

Beispiel 1

8,1 Gewichtsteile p-Chlorbenzylchlorid und 13,2 Gewichtsteile N-[3-Trifluormethyl-4-hydroxyphenyl]-N'-methyl-N'-methoxyharnstoff werden mit 7,0 Gewichtsteilen Kaliumcarbonat in 200 Volumenteilen Acetonitril 8 Stunden unter Rückfluß erhitzt. Anschließend läßt man abkühlen, gibt in Eiswasser und saugt ab. Man erhält 18,9 Gewichtsteile N-[3-Trifluormethyl-4-(4-chlorbenzyloxy)-phenyl]-N'-methyl-N'-methoxyharnstoff vom Schmelzpunkt 135 bis 137°C (Verbindung Nr. 1).

Beispiel 2

122 Gewichtsteile 2-Phenylethanol werden mit 225 Gewichtsteilen 3-Trifluormethyl-4-chlor-nitrobenzol und 138 Gewichtsteilen Kaliumcarbonat unter Zugabe von 10 g Tetrabutylammoniumjodid 19 Stunden in 200 Volumenteilen Dimethylformamid unter Rückfluß erhitzt. Anschließend wird gekühlt, abfiltriert und das Filtrat eingeengt. Den verbleibenden Rückstand

verrührt man in wenig Methanol und saugt wiederum ab. Man erhält 176 Gewichtsteile 3-Trifluormethyl-4-(2-phenylethoxy)-nitrobenzol vom Schmelzpunkt 50 bis 52°C.

80 Gewichtsteile 3-Trifluormethyl-4-(2-phenylethoxy)-nitrobenzol werden in 100 Volumenteilen Ethanol und 125 Volumenteilen conc. Salzsäure suspendiert und bei 50°C portionsweise mit 221 Gewichtsteilen Zinn-(II)-chlorid versetzt. Anschließend wird 4 Stunden bei 50 bis 60°C nachgerührt, in Eiswasser eingerührt, mit Natronlauge alkalisch gemacht und das gewünschte Produkt mit Methylenchlorid extrahiert. Das nach Abdampfen des Methylenchlorids erhaltene Öl wird mit 10 %iger Salzsäure verrührt und abgesaugt. Man erhält 79,8 Gewichtsteile 3-Trifluormethyl-4-(2-phenylethoxy)-anilinhydrochlorid vom Schmelzpunkt 185 bis 187°C.

15,9 Gewichtsteile 3-Trifluormethyl-4-(2-phenylethoxy)-anilinhydrochlorid werden in 100 Volumenteilen Aceton suspendiert. Nach Zugabe einer Lösung von 8,4 g Natriumhydrogencarbonat in 100 Volumenteilen Wasser wird auf 5°C gekühlt und tropfenweise mit 6,2 Gewichtsteilen N-Methyl-N-methoxycarbaminsäurechlorid versetzt. Nach mehrstündigem Nachrühren bei 5°C wird in Eiswasser gegeben und mit Methylenchlorid extrahiert. Das erhaltene Öl wird in Cyclohexan verrührt und nach Kristallisation abgesaugt. Man erhält 12,2 Gewichtsteile N-[3-Trifluormethyl-4-(2-phenylethoxy)-phenyl]-N'-methyl-N'-methoxyharnstoff vom Schmelzpunkt 57 bis 58°C (Verbindung Nr. 2).

Entsprechend können die folgenden Harnstoffe der Formel I hergestellt werden.

| Verbindung Nr. | X | $Y_m$ | n | Fp. [°C]/$n_D$ |
|---|---|---|---|---|
| 1 | $-CH_2-$ | 4-Cl | 0 | 135 - 137 |
| 2 | $-CH_2CH_2-$ | H | 0 | 57 - 58 |
| 3 | $-CH_2-$ | 3-Cl | 0 | 85 - 86 |
| 4 | $-CH_2-$ | $4-CH_3$ | 0 | 128 - 129 |
| 5 | $-CH_2-$ | H | 0 | 91 - 93 |
| 6 | $-CH(CH_3)-$ | H | 0 | |
| 7 | $-CH_2-$ | 4-F | 0 | 124 - 126 |
| 8 | $-CH_2-$ | 3-F | 0 | 75 - 77 |
| 9 | $-CH_2-$ | $3-CH_3$ | 0 | |
| 10 | $-CH_2CH_2-$ | $4-CH_3$ | 0 | Öl $n_D^{22}$: 1,5357 |
| 11 | $-CH_2CH_2-$ | 4-Cl | 0 | |
| 12 | $-CH_2C{\equiv}C-CH_2-$ | H | 1 | 83 - 85 |
| 13 | $-CH_2CH_2CH_2-$ | H | 1 | |
| 14 | $-CH_2CH_2-$ | H | 1 | |
| 15 | $-CH_2CH_2CH_2-$ | 4-Cl | 1 | |
| 16 | $-CH_2CH_2CH_2CH_2-$ | H | 1 | |
| 17 | $-CH_2CH=CH-CH_2-$ | H | 1 | 92 - 94 |
| 18 | $-CH_2CH=CH-CH_2-$ | H | 0 | |
| 19 | $-CH_2CH_2CH_2-$ | H | 0 | zähes Öl |
| 20 | $-CH_2CH_2-$ | $2-CH_3$ | 1 | 100 - 102 |
| 21 | $-CH_2CH_2CH_2-$ | 3-Cl | 1 | 80 - 82 |
| 22 | $-CH_2CH_2CH_2CH_2-$ | $3,4-Cl_2$ | 1 | 85 - 87 |
| 23 | $-CH=CH-CH_2-$ | H | 0 | |
| 24 | $-CH_2-$ | $3-CF_3$ | 0 | 103 - 104 |
| 25 | $-CH_2-$ | $2,4-Cl_2$ | 0 | 140 - 143 |

Die Harnstoffderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-$\alpha$-pyrrolidon und erhält eine Lösung, die zur Anwendung in Form kleinster Tropfen geeignet ist.

II. 10 Gewichtsteile der Verbindung Nr. 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono--ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 5 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 5 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an

1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 2 werden mit 3 Gewichtsteilen des Natriumsalzes der Diisobutyl-naphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 10 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 10 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 21 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der sie enthaltenden herbiziden Mittel kann im Vorauflauf- oder im Nachauflaufverfahren erfolgen. Vorzugsweise werden sie nach dem Auflaufen der unerwünschten Pflanzen ausgebracht. Sind die Wirkstoffe bei Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadien, Bodenart und Anwendungsverfahren 0,1 bis 3, vorzugsweise 0,125 bis 1,0 kg/ha.

Die herbizide Wirkung der Harnstoffderivate der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät.

Zum Zwecke der Nachauflaufanwendung zieht man die Testpflanzen je nach Wuchsform bis zu einer Wuchshöhe von 3 bis 15 cm an und behandelt sie danach. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,125 und 0,5 kg Wirkstoff/ha. Zum Aufbringen der Wirkstoffe werden diese in Wasser als Verteilungsmittel suspendiert oder emulgiert und mittels fein verteilender Düsen gespritzt.

Die Versuchsgefäße werden im Gewächshaus augestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Amaranthus retroflexus (Zurückgekrümmter Fuchsschwanz), Cassia tora, Cirsium arvense (Acker-Kratzdistel), Euphorbia geniculata (Südamerik. Wolfsmilchart), Galium aparine (Klettenlabkraut), Ipomoea spp. (Prunkwindearten), Solanum nigrum (Schwarzer Nachtschatten), Triticum aestivum (Weizen).

Vergleichsmittel sind die aus DE-OS 27 11 230 bekannten herbiziden Harnstoffe N-[4-Benzyloxyphenyl]-N'-methoxy-N'-methylharnstoff (A) und N-[4--Benzyloxy-3-chlorphenyl]-N'-methoxy-N'-methylharnstoff (B). Die Aufwandmengen an Vergleichswirkstoffen entsprechen denjenigen an erfindungsgemäßen Verbindungen.

Bei Nachauflaufanwendung zeigt beispielsweise Verbindung Nr. 5 mit 0,5 kg Wirkstoff/ha eine gute herbizide Wirkung. Das Vergleichsmittel A wirkt dagegen sehr schwach. Auch die Verbindung Nr. 2 ist in ihrer herbiziden Wirkung sowohl der Vergleichssubstanz A als auch der Vergleichssubstanz B

mit 0,125 kg Wirkstoff weit überlegen. Beide erfindungsgemäßen Verbindungen sind selektiv in Weizen.

In Anbetracht der Verträglichkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewisse Standorten unerwünscht sind, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napobrassica | Kohlrübe |
| Camellia sinensis | Teestrauch |
| Carthamus tinctorius | Saflor - Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Elaeis guineensis | Ölpalme |
| Fragaria vesca | Erdbeere |
| Glyoine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |

| Botanischer Name | Deutscher Name |
|---|---|
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolben-hirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |

| Botanischer Name | Deutscher Name |
|---|---|
| Theobroma cacao | Kakaobaum |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Harnstoffderivate der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, halogencarbonsäuren, Triazine, Amide, andere Harnstoffe, Triazinone, Uracile, Benzofuranderivate, Cyclohexan-1,3-dion-derivate und andere in Betracht.

Außerdem kann es von Nutzen sein, die neuen Verbindungen allein oder in Kombination mit anderen Herbiziden auch noch mit weiteren Pflanzenschutzmitteln gemischt geminsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

Patentansprüche

1. Harnstoffderivate der Formel

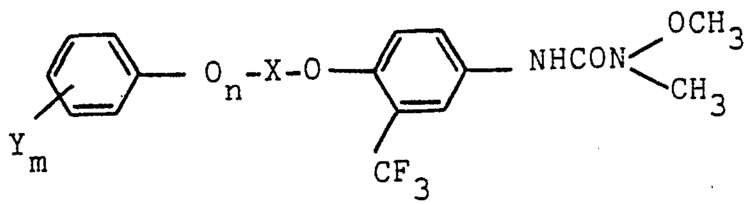 (I),

wobei

X eine gegebenenfalls durch $C_1$-$C_4$-Alkyl substituierte $C_1$-$C_4$-Alkylenkette, $C_2$-$C_4$-Alkenylenkette oder $C_2$-$C_4$-Alkinylenkette,

Y Wasserstoff, $C_1$-$C_4$-Alkyl, Halogen, $C_1$-$C_4$-Alkoxy oder $C_1$-$C_4$-Halogenalkyl,

m 1 bis 3 und

n 0 oder 1

bedeuten.

2. Harnstoffderivate der Formel I gemäß Anspruch 1, dadurch gekennzeichnet, daß X eine $C_1$-$C_4$-Alkylenkette, Y Wasserstoff und n 0 bedeuten.

3. Verfahren zur Herstellung von Harnstoffderivaten der Formel I gemäß Anspruch I, dadurch gekennzeichnet, daß man eine Halogenverbindung der Formel II

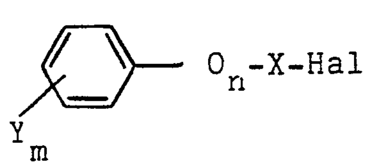 (II),

in der

X, Y, n und m die im Anspruch 1 genannten Bedeutungen haben und Hal für Halogen steht, N-(4-Hydroxy-3-trifluormethyl-phenyl), N'-methyl-N'-methoxy-harnstoff in Gegenwart eines inerten Lösungsmittels bei einer Temperatur im Bereich zwischen 0 und 150°C umsetzt.

4. Verfahren zur Herstellung von Harnstoffderivaten der Formel I gemäß Anspruch 1, dadurch gekennzeichnet, daß man Hydroxylverbindungen der Formel

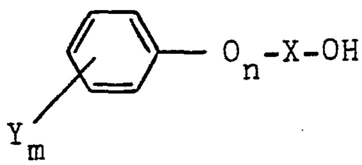

(III),

in der Y, X, m und n die im Anspruch 1 genannten Bedeutungen haben, mit einer Halogenverbindung der Formel

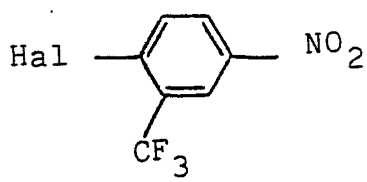

(IV),

in der Hal Halogen bedeutet, umsetzt, die so entstandene Nitroverbindung in an sich üblicher Weise zur Aminoverbindung reduziert und diese mit N-Methyl-N'-methoxy--carbaminsäurehalogenid in das Harnstoffderivat der Formel I überführt.

5. Herbizid, enthaltend ein Harnstoffderivat der Formel I gemäß Anspruch 1.

6. Herbizid, enthaltend inerte Zusatzstoffe und ein Harnstoffderivat der Formel I gemäß Anspruch 1.

7. Herbizid nach Anspruch 6, <u>dadurch gekennzeichnet</u>, daß es 0,1 bis 95 Gew.% Harnstoffderivat der Formel I enthält.

8. Herbizid nach Anspruch 6, <u>dadurch gekennzeichnet</u>, daß es ein Harnstoffderivat der Formel I enthält, wobei X eine $C_1$-$C_4$-Alkylenkette, Y Wasserstoff und n 0 bedeuten.

9. Verfahren zur Bekämpfung unerwünschten Pflanzenwuchses, <u>dadurch gekennzeichnet</u>, daß man die unerwünschten Pflanzen oder die von unerwünschtem Pflanzenwuchs freizuhaltende Fläche mit einer herbizid wirksamen Menge eines Harnstoffderivates der Formel I gemäß Anspruch 1 behandelt.

10. Verfahren nach Anspruch 9, <u>dadurch gekennzeichnet</u>, daß die Menge an Harnstoffderivat der Formel I 0,1 bis 3 kg/ha beträgt.

Europäisches Patentamt

# EUROPÄISCHER RECHERCHENBERICHT

0133918

Nummer der Anmeldung

EP 84 10 7710

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | US-A-4 289 903 (D.M. SPATZ u.a.) * Ansprüche; Spalten 7-8 * --- | 1-10 | C 07 C 127/19<br>A 01 N 47/30 |
| A | DE-A-1 918 114 (CIBA) * Ansprüche * --- | 1-10 | |
| A | GB-A-2 000 766 (SUMITOMO CHEM.) * Ansprüche * ----- | 1-10 | |
| | | | RECHERCHIERTE SACHGEBIETE (Int. Cl. 3) |
| | | | C 07 C 127/00 |

Der vorliegende Recherchenbericht wurde für alle Patentansprüche erstellt.

| Recherchenort | Abschlußdatum der Recherche | Prüfer |
|---|---|---|
| DEN HAAG | 15-10-1984 | GAUTIER R.H.A. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82